# EUROPEAN PATENT APPLICATION

(11) **EP 4 431 158 A1**
(43) Date of publication of application: **18.09.2024**
(21) Application number: 22890059.3
(22) Date of filing: 07.11.2022
(51) Int. Cl.: A61Q 1/00, A61Q 1/02, A61Q 1/10, A61K 8/02, A61K 8/06, A61K 8/19, A61K 8/24, A61K 8/34, A61K 8/81, A61K 8/85, A61K 8/88

(54) **AQUEOUS LIQUID COSMETIC**

(30) Priority: 08.11.2021 JP 2021182049
(71) Applicant: Mitsubishi Pencil Company, Limited, Tokyo 140-8537 (JP)
(72) Inventor: SATO Moe, Fujioka-shi, Gunma 375-8501 (JP); INOUE Kensuke, Fujioka-shi, Gunma 375-8501 (JP)
(74) Representative: Vossius & Partner Patentanwälte Rechtsanwälte mbB
(86) International application number: PCT/JP2022/041410
(87) International publication number: WO 2023/080239

(57) **Abstract**

Provided is an aqueous liquid cosmetic which does not cause aggregation and sedimentation of colorant particles even when the cosmetic contains a colorant composed only of an inorganic pigment such as an iron oxide pigment, which can highly improve the density of a drawn line, which can also improve fixation, and which is suitable as a makeup cosmetic such as an eyeshadow, an eyeliner, an eyebrow cosmetic, or a mascara. The aqueous liquid cosmetic of the first disclosure is a cosmetic stored in a pen-type applicator having a brush at an application portion, and contains at least an iron oxide pigment, a polyorganic acid or a polyorganic acid salt, emulsion particles containing acrylateacrylate copolymer, a nonionic surfactant, and an aqueous solvent.

## Description

### Technical Field

The present specification relates to an aqueous liquid cosmetic suitable as a makeup cosmetic such as an eyeshadow, an eyeliner, an eyebrow cosmetic, or a mascara.

### Background Art

In the related art, Patent Document 1 discloses an aqueous liquid cosmetic containing, as a composition of a cosmetic suitable as an eyeliner, diethylhexyl sodium sulfosuccinate, an inorganic coloring pigment, a water-soluble dispersant, and a film-forming polymer emulsion, wherein the content of the diethylhexyl sodium sulfosuccinate is 0.01 to 1 mass% relative to the total amount of the cosmetic, the content of the inorganic coloring pigment is 3 to 20 mass% relative to the total amount of the cosmetic, and the inorganic coloring pigment contains carbon black. The document discloses that the film-forming polymer emulsion contains a core-shell-type alkyl acrylate copolymer emulsion, and such a composition having a low viscosity is stored in an applicator called a collector-type applicator.

Patent Document 1 describes that dispersion is sufficiently performed with a water-soluble resin to form a cosmetic film having excellent density of drawn lines and good uniformity by using carbon black in an amount of half or more of the weight of the pigment. It can be seen that the sedimentation of the pigment is suppressed as much as possible by increasing the proportion of carbon black having a specific gravity smaller than that of the iron oxide pigment. In this case, when another inorganic pigment having a large specific gravity (such as iron oxide) is used in combination, a problem of so-called color separation tends to occur, and a uniform drawn line cannot be drawn, and thus further improvement has been desired.

Patent Document 2 describes that a makeup cosmetic such as an eyeliner contains (A) sodium polyaspartate, (B) at least one bright powder selected from Group B1: metal or metal oxide-coated glass powder, aluminum powder, Group B2: metal-coated film powder, (C) a water-soluble dispersant, and (D) at least one metal oxide selected from Group D1 (yellow iron oxide, red iron oxide, black iron oxide, titanium dioxide, titanium oxynitride) and Group D2 (iron blue), and that another water-soluble polymer is added as a dispersant, in order to obtain bright drawn lines and to improve redispersion ability of a bright pigment. However, there is a problem that "good fixation" in Patent Document 2 means that smear caused by rubbing is reduced from an estimated level of higher than 20% to a level below 20% and 10% or higher, and it cannot be said that this level is sufficient.

Meanwhile, as a typical makeup cosmetic composition, Patent Document 3 discloses an emulsion-type composition containing water and a lower polyhydric alcohol in an aqueous phase, and further containing an inorganic pigment such as iron oxide, a core-shell-type emulsion of an acrylic polymer, a hydrating oil, and a wax. This makeup cosmetic can be washed away with warm water while exhibiting a certain water resistance.

However, the cosmetic described in Document 3 contains a hydrating oil as an essential component in exchange for the fact that it can be washed away with warm water. Therefore, even if it is dried after coating, moisture remains in the coating film, and even if it has water resistance, there is a possibility that the fixation will be dissolved by moisture such as sweat or tears.

Meanwhile, in recent years, in consideration of skin, "surfactant-free" (a composition free from a surfactant) makeup cosmetics are also increasing in the market in response to consumer's needs to avoid synthetic surfactants as much as possible.

### Citation List

### Patent Document

Patent Document 1: JP 2020-70259 A (Claims, Examples, etc.)
Patent Document 2: JP 2020-132619 A (Claims, Examples, etc.)
Patent Document 3: JP 2010-265225 A (Claims, Examples, etc.)

### Summary of Invention

### Technical Problem

The first disclosure is intended to solve the problems and current situation with regard to the aqueous liquid cosmetics of, for example,

Patent Documents 1 and 2 described above as the related art. An object of the first disclosure is to provide an aqueous liquid cosmetic which does not cause aggregation and sedimentation of colorant particles even when it contains a colorant composed only of an inorganic pigment such as an iron oxide pigment, which can improve the density of drawn lines, which can also improve fixation, and which is suitable as a makeup cosmetic such as an eyeshadow, an eyeliner, an eyebrow cosmetic, or a mascara.

The second disclosure is intended to solve the problems and current situation with regard to the aqueous liquid cosmetics of Patent Documents 1 and 3 described above as the related art. An object of the second disclosure is to provide an aqueous liquid cosmetic which contains a pigment such as carbon black as a colorant, which can highly achieve fixation and cleansing properties of drawn lines even if it does not contain a surfactant, and which is suitable as a makeup cosmetic such as an eyeshadow, an eyeliner, an eyebrow cosmetic, or a mascara. Solution to Problem

In consideration of the typical problems of Patent Documents 1 and 2 mentioned above and as a result of diligent studies, the present inventors have found a cosmetic stored in a pen-type applicator having a brush at an application portion, that achieves the above object can be obtained in the form of an aqueous liquid cosmetic having a composition containing at least an iron oxide pigment, a polyorganic acid or a polyorganic acid salt, specific emulsion particles, a specific surfactant, and an aqueous solvent, and have completed the first disclosure.

Further, in consideration of the typical problems of Patent Documents 1 and 3 mentioned above and as a result of diligent studies, the present inventors have found a cosmetic stored in an applicator of a type in which liquid is fed from an application liquid storage portion to an application portion using capillary force, that achieves the above object can be obtained in the form of an aqueous liquid cosmetic containing at least a pigment containing carbon as a main component, a specific water-soluble acrylic copolymer, core-shell-type emulsion particles containing acrylate copolymer, and an aqueous solvent, and have completed the second disclosure.

That is, the aqueous liquid cosmetic of the first disclosure is a cosmetic stored in a pen-type applicator having a brush at an application portion, and contains at least an iron oxide pigment, a polyorganic acid or a polyorganic acid salt, emulsion particles containing acrylate copolymer, a nonionic surfactant, and an aqueous solvent.

The polyorganic acid is preferably at least one selected from Group A below.

Group A: polyaspartic acid, polylactic acid, polyphosphoric acid, and polyacrylic acid.

The emulsion particles containing acrylate copolymer are preferably core-shell-type emulsion particles.

The aqueous solvent is preferably selected from water, a lower alcohol having 5 or less carbons, or a polyhydric alcohol.

Further, it is preferable to contain an inorganic pigment other than the iron oxide pigment.

The aqueous liquid cosmetic of the second disclosure is a cosmetic stored in an applicator having a type in which liquid is fed from an application liquid storage portion to an application portion using capillary force, the cosmetic containing at least a pigment containing carbon as a main component, a water-soluble acrylic copolymer selected from Group X below, core-shell-type emulsion particles containing acrylate copolymer, and an aqueous solvent.

Group X: (styrene/acrylate) copolymer and ammonium acrylate copolymer. The pigment containing carbon as a main component is preferably selected from carbon black and/or graphite.

The aqueous solvent is preferably selected from water, a lower alcohol having 6 or less carbons, or a polyhydric alcohol.

The content of water is preferably 50 mass% or more.

The application portion of the applicator is preferably composed of a brush.

Further, it is preferable to contain an inorganic pigment other than the iron oxide pigment.

As used herein, the term "aqueous" refers to a composition of a single system (not a W/O emulsion, an O/W emulsion, or the like) containing water and/or a water-soluble organic solvent as a main solvent. In addition, "the present disclosure" means that the disclosed contents of both the aqueous liquid cosmetic of the first disclosure and the aqueous liquid cosmetic of the second disclosure are included.

### Advantageous Effects of Invention

According to the first disclosure, there is provided an aqueous liquid cosmetic which does not cause aggregation and sedimentation of colorant particles even if it contains a colorant composed only of an inorganic pigment such as an iron oxide pigment, which can improve the density of drawn lines, which can also improve fixation, and which is suitable as a makeup cosmetic such as an eyeshadow, an eyeliner, an eyebrow cosmetic, or a mascara.

According to the second disclosure, there is provided an aqueous liquid cosmetic which contains a pigment such as carbon black as a colorant, which can highly achieve fixation and cleansing properties of drawn lines even if it does not contain a surfactant, and which is suitable as a makeup cosmetic such as an eyeshadow, an eyeliner, an eyebrow cosmetic, or a mascara.

The object and effects of the present disclosure can be recognized and obtained especially using the components and combinations indicated in the claims. Both general explanation described above and detailed explanation described below are exemplary and explanatory and do not limit the present disclosure described in claims.

### Brief Description of Drawings

FIG. 1 is a partial cross-sectional view illustrating an example of a liquid cosmetic applicator that stores an aqueous liquid cosmetic of the first disclosure. The mechanism illustrated in FIG. 1 is an example of a rotary extension type.
FIG. 2 is a partial cross-sectional view illustrating another example of a liquid cosmetic applicator that stores the aqueous liquid cosmetic of the first disclosure. The mechanism illustrated in FIG. 2 is an example of a type of applicator with built-in cosmetic.
FIG. 3(a) is a perspective view illustrating still another example of a liquid cosmetic applicator that stores the aqueous liquid cosmetic of the first disclosure, and FIG. 3(b) is a partial cross-sectional view of the same liquid cosmetic applicator as illustrated in FIG. 3 (a) .
The mechanism of the liquid cosmetic applicator of FIGS. 3(a) and 3(b) is an example of a collector-type applicator.
FIG. 4 is a vertical cross-sectional view illustrating an example of a liquid cosmetic applicator that stores an aqueous liquid cosmetic of the second disclosure and illustrating a state before use.
FIG. 5 is a vertical cross-sectional view illustrating the liquid cosmetic applicator of FIG. 4 at the start of use.
FIG. 6 is a partial vertical cross-sectional view illustrating another example of a liquid cosmetic applicator that stores the aqueous liquid cosmetic of the second disclosure.
FIG. 7 is a partial cross-sectional view illustrating another example of a liquid cosmetic applicator that stores the aqueous liquid cosmetic of the second disclosure.

### Description of Embodiments

Embodiments of the present disclosure will be described below in detail. However, it should be noted that the technical scope of the present disclosure is not limited to the embodiments detailed below and includes the invention described in claims and equivalents thereof. In addition, the present disclosure can be implemented based on the contents disclosed in the present specification and technical common knowledge (including design matters and obvious matters) in the art.

### Aqueous Liquid Cosmetic of First Disclosure

The aqueous liquid cosmetic of the first disclosure is a cosmetic stored in a pen-type applicator having a brush at an application portion, and contains at least an iron oxide pigment, a polyorganic acid or a polyorganic acid salt, emulsion particles containing acrylate copolymer, a nonionic surfactant, and an aqueous solvent.

Examples of the iron oxide pigment used in the first disclosure include at least one (alone or a mixture of two or more types, the same shall apply hereinafter) of black iron oxide, red ocher (red iron oxide), or yellow iron oxide. The particle shape of such an iron oxide pigment is not particularly limited and may be any shape such as a spherical, granular, rod-like, needle-like, plate-like, or amorphous shape.

In addition, the average particle size of the iron oxide pigment (particles) that can be used before dispersion (at the time of blending) can vary depending on the type of the iron oxide pigment. For example, in the case of red ocher, black iron oxide, or yellow iron oxide, particles having an average particle size of 2000 nm or less, preferably 100 to 1000 nm can be used.

According to the first disclosure, among the iron oxide pigments described above, when red ocher, which has a high specific gravity and is a colorant useful for eye cosmetics such as a brown eyeliner, is used excellent dispersibility is achieved, and no color separation is exhibited, which are the intended effect.

In the present disclosure (including Examples), the "average particle size" refers to a value measured and calculated by a dynamic light scattering method [particle size analyzer FPAR-1000, available from Otsuka Electronics Co., Ltd.].

The content of the iron oxide pigment is preferably 0.3 to 20 mass%, and more preferably 0.3 to 15 mass%, relative to the total amount of the cosmetic composition.

When the content of the iron oxide pigment is less than 0.3 mass%, the color development is insufficient and the concealing ability is also insufficient, whereas when the content exceeds 20 mass%, the viscosity is increased and writing is hindered, which is not preferred.

As the inorganic pigment other than the iron oxide pigment, at least one of carbon black, iron blue, titanium oxide, titanium black, zinc oxide, talc, chromium oxide, cobalt oxide, zinc oxide, titanium mica, Red No. 226, bismuth oxychloride, ultramarine, or ferric ferrocyanide can be used.

Such an inorganic pigment is used as a colorant, and when carbon black is selected, the color can be adjusted to black or brown by using the carbon black in combination with the iron oxide pigment. The usable inorganic pigment including carbon black can be used without any limitation as long as it is used in liquid cosmetics. Such an inorganic pigment may also have an average particle size of 2000 nm or less, preferably 100 to 1000 nm.

The content of the inorganic pigment other than the iron oxide pigment is preferably 0 to 15 mass% relative to the total amount of the cosmetic composition, and is about 0.1 to 3 mass% when the color is adjusted to, for example, a brown color or a black color by using the inorganic pigment in combination with the iron oxide pigment without using titanium oxide.

The polyorganic acid and the polyorganic acid salt used in the first disclosure improve the dispersibility of the iron oxide pigment serving as a colorant, and examples thereof include at least one selected from the following Group A.

Group A: polyaspartic acid, polylactic acid, polyphosphoric acid, and polyacrylic acid.

Examples of the polyorganic acid salt to be used include salts of polyaspartic acid, polylactic acid, polyphosphoric acid, and polyacrylic acid of Group A described above. The cosmetic may contain at least any one or more of these salts. Examples of the types of the salts thereof include alkali metal salts, alkaline earth metal salts, ammonium salts, and alkanolamine salts. Sodium salts, potassium salts, and lithium salts are particularly preferred.

Among these polyorganic acids or polyorganic acid salts, polyaspartic acid or a salt thereof is particularly excellent in dispersion stability, and sodium polyaspartate is particularly preferred.

The (total) content of the polyorganic acid or the polyorganic acid salt is preferably 0.2 to 2.0 mass% and more preferably 0.4 to 1.85 mass%, relative to the total amount of the liquid cosmetic.

When the content of the polyorganic acid or the polyorganic acid salt is 0.2 mass% or more, the dispersion stability of the iron oxide pigment such as red ocher serving as a colorant is improved, whereas when the content thereof is 2.0 mass% or less, a good viscosity range can be achieved, and the dispersion stability is improved.

The emulsion particles used in the first disclosure are film-forming polymer emulsion particles and contain acrylate copolymer, and an aqueous dispersion of a water-insoluble polymer can be used. Examples of the emulsion particles that can be used include a styrene-alkyl acrylate copolymer emulsion, a styrene-alkyl methacrylate copolymer emulsion, and a copolymer emulsion containing a component of acrylic acid, methacrylic acid, or (C1 to C4 and C8) alkyl ester thereof.

It may be an emulsion of a composite polymer such as a core-shell-type polymer emulsion containing a copolymer of an ethylenically unsaturated carboxylic acid monomer and a styrene monomer and another polymer and/or copolymer.

Specific examples of the styrene-alkyl acrylate copolymer emulsion include (styrene/acrylate) copolymer. Examples of commercially available products of this copolymer include YODOSOL GH41F (available from Nouryon) and DAITOSOL 5000STY (available from DAITO KASEI KOGYO CO., LTD.).

Specific examples of the emulsion of the core-shell-type polymer emulsion containing a copolymer of an ethylenically unsaturated carboxylic acid monomer and a styrene monomer and another polymer and/or copolymer include EMUPOLY-CE-119N (available from Gifu Shellac Mfg. Co., Ltd.), which is an emulsion of core-shell-type copolymer of ethylhexyl acrylate/methacrylic acid copolymer [(acrylate/methylstyrene/styrene) copolymer ammonium].

From the viewpoint of film-forming properties and fixation of drawn lines, it is preferable to contain acrylate copolymer, and more preferably, it is desirable to use the core-shell-type polymer emulsion.

In such emulsion particles, a resin component is usually finely dispersed in an aqueous component at a concentration of 20 to 60 mass% as a solid content. The blended amount of the emulsion particles is 1 to 30 mass%, preferably 5 to 15 mass% in view of solid content concentration relative to the entire liquid cosmetic.

When the content of the emulsion particles is less than 1 mass%, the fixation and the water-resistant fixation are lowered, whereas when the content thereof is more than 30 mass%, the viscosity of the liquid cosmetic is increased, leading to difficulty in application of the cosmetic.

Examples of the nonionic surfactant used in the first disclosure include, particularly, a surfactant in which polyoxyethylene (POE) and an organic acid, glycerin, or a higher alcohol are bound via an ether bond, more preferably, polyoxyethylene (POE) alkyl ethers such as laureth (polyoxyethylene lauryl ether), ceteth (polyoxyethylene cetyl ether), steareth (polyoxyethylene stearyl ether), and beheneth (polyoxyethylene behenyl ether). These can be used alone or in combination of two or more.

Among them, in view of the molecular structure and hydrophilicity, it is preferable to use POE (20) cetyl ether (ceteth-20), POE (30) behenyl ether (beheneth-30), POE (20) stearyl ether, or POE (10) oleyl ether.

The content of the nonionic surfactant to be used is preferably 0.01 to 1 mass%, and more preferably 0.03 to 0.5 mass%, relative to the total amount of the liquid cosmetic, from the viewpoint of preventing excessive penetration into a skin (so-called spider) and from the viewpoint of dispersion stability of the iron oxide pigment.

The aqueous solvent used in the first disclosure is used as a solvent of a liquid cosmetic. Examples thereof include at least one (alone or a mixture of two or more types, the same shall apply hereinafter) of water, a lower alcohol having 5 or less carbons, or a polyhydric alcohol. Specific examples of the lower alcohol having 5 or less carbons include at least one of methyl alcohol (methanol), ethyl alcohol (ethanol), n-propylalcohol, isopropyl alcohol, n-butylalcohol, sec-butylalcohol, tert-butyl alcohol, isobutyl alcohol, pentyl alcohol, ethylene glycol, or propylene glycol.

Examples of the polyhydric alcohol include glycerin, erythritol, threitol, arabinitol, xylitol, ribitol, propylene glycol, 1,3-butylene glycol, 1,2-pentanediol, ethylene glycol, diethylene glycol, and pentaerythritol.

In particular, it is desired to use ethyl alcohol (ethanol), from the perspective of safety and handleability.

The total content of the aqueous solvent to be used is preferably 45 to 85 mass%, and more preferably 50 to 80 mass%, relative to the entire amount of the liquid cosmetic, in view of safety and dispersion stability of the iron oxide pigment, and particularly in view of stability at low temperatures.

When the content of the aqueous solvent is 45 mass% or more, smooth writing and moisture retaining properties can be achieved, and an antiseptic effect can be slightly achieved. Meanwhile, when the content thereof is 85 mass% or less, the dispersion stability is further improved.

Water serving as a solvent used in the first disclosure can be distilled water, ion-exchanged water, purified water, pure water, or ultrapure water. In view of further improving stability over time and dispersibility of the iron oxide pigment, the ratio of water to the aqueous solvent (water + a solvent other than water) is preferably 70 to 100 mass% (50 to 80 mass%), and particularly preferably 80 to 90 mass%.

Further, the aqueous liquid cosmetic of the first disclosure may contain, in addition to the above-described components, an optional component used in ordinary liquid cosmetics. Specifically, the aqueous liquid cosmetic may contain an appropriate amount of a preservative, an antioxidant, a neutralizing agent, an ultraviolet absorber, a chelating agent, a pH adjuster, a humectant, a beauty ingredient, a perfume, a viscosity/stickiness modifier, or another dispersant such as polyethylene glycol alkyl ether, as long as the effects of the first disclosure are not impaired. Besides the inorganic pigment other than the iron oxide pigment, an additional colorant such as an organic pigment, a water-soluble dye, or a resin particle pigment may optionally be contained, as long as the effects of the present disclosure are not impaired.

The aqueous liquid cosmetic of the first disclosure preferably has a viscosity of 15 mPa·s or less, preferably 10 mPa·s or less, and more preferably 2 to 8 mPa·s.

When the viscosity exceeds 15 mPa·s, the flowability of the aqueous liquid cosmetic is significantly decreased, which is not preferred. Adjustment of the above viscosity range can be performed, for example, by suitably combining the respective components such as the iron oxide pigment type to be used and its average particle size, the polyorganic acid or polyorganic acid salt, the emulsion particles containing acrylate copolymer, the nonionic surfactant, and the aqueous solvent, by combining the contents of the respective components within a suitable range, or by a suitable dispersion method.

In the first disclosure, regarding the viscosity measurement conditions (including Examples described below), specifically, the resultant cosmetic composition is subjected to measurement with an ELD-type viscometer available from Toki Sangyo Co., Ltd using a standard rotor: 50 rpm (shear rate: 192 [s-1]) at a temperature of 25°C.

The aqueous liquid cosmetic of the first disclosure preferably has a pH (25°C) in a range of 6 to 9 when measured using a glass electrode, from the viewpoint of improving solubility and suppressing skin irritation. The pH is adjusted using the above-mentioned pH adjuster or the like.

When the aqueous liquid cosmetic of the first disclosure is prepared by using the above-mentioned iron oxide pigment, polyorganic acid or polyorganic acid salt, emulsion particles containing acrylate copolymer, nonionic surfactant, aqueous solvent, and additional component under suitable dispersion conditions using, for example, a dispersing machine such as a homomixer, a sand mill, an ultrasonic homogenizer, or a high-pressure homogenizer, the average particle size of the iron oxide pigment in the cosmetic (after dispersion) can be adjusted to 300 nm or less.

Preferably, when a (multiple type) ultrasonic homogenizer or a high-pressure homogenizer is used as the dispersion machine, the average particle size of the iron oxide pigment in the cosmetic (after dispersion) can be adjusted to 300 nm or less by setting the dispersion conditions; for example, the frequency is set to 20 to 30 KHz in the case of an ultrasonic homogenizer, or the pressure is set to 150 to 245 MPa in the case of a high-pressure homogenizer.

The resultant aqueous liquid cosmetic can be stored in a pen-type applicator having a brush as an application portion. For example, the aqueous liquid cosmetic can be stored (charged) in a pen-type liquid cosmetic applicator (container) having a brush or a pen core as an application portion for use.

The liquid cosmetic applicator that can be used is not particularly limited as long as it is a liquid cosmetic applicator equipped with a brush or a pen core for, for example, an eyeliner or an eyebrow pen. Preferred examples of the liquid cosmetic applicator include an applicator having, as application means, a brush (brush pen) or a pen core for an eyeliner or an eyebrow pen, or an application body formed of a rubber, an elastomer, or a closed-cell foam having restorability, and having a container for charging the liquid cosmetic.

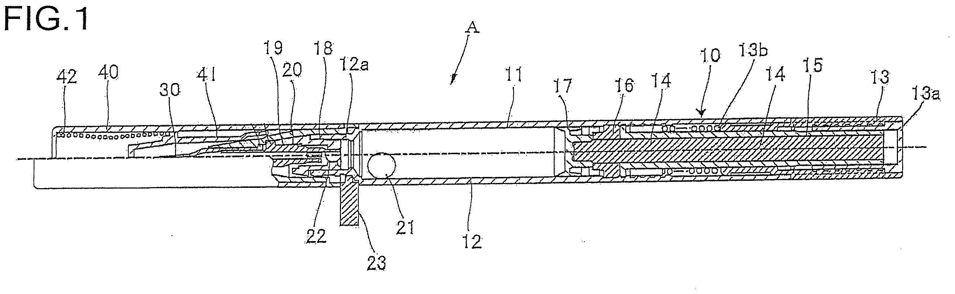

Specifically, as illustrated in FIG. 1, FIG. 2, FIG. 3(a), and 3b, it is desirable to use a liquid cosmetic applicator which is excellent in usability, convenience, and application properties and has a mechanism of a rotary extension type, a mechanism of a type of applicator with built-in cosmetic, or a mechanism of a collector-type applicator.

As illustrated in FIG. 1, a liquid cosmetic applicator A of a rotary extension type includes an application portion 30 formed of a brush (brush pen) provided in front of a cosmetic container 11 serving as a reservoir configured to discharge, by a liquid pressing mechanism 10, the liquid cosmetic of the present disclosure (hereinafter referred to simply as "liquid cosmetic") contained in front of the liquid pressing mechanism 10.

The liquid pressing mechanism 10 has such a configuration that a feeding member 13 disposed at a rear end portion of a barrel body 12 is relatively rotated with respect to the barrel body 12 in a circumferential direction to feed the liquid cosmetic in the container (reservoir) 11, and then the liquid cosmetic is supplied to the application portion 30.

The liquid pressing mechanism 10 of the applicator has the feeding member 13 rotatably fitted to the rear end of the barrel body 12; a driving cylinder 15 configured to transmit the rotational force of the feeding member 13 by a user to a screw rod 14; a screw body 16 fixed to the barrel body 12 and screwed with the screw rod 14; the screw rod 14 rotatably engaged with a piston body 17 at its tip; and the piston body 17 configured to slide in the reservoir 11 of the barrel body 12. The rotation of the feeding member 13 is transmitted to the screw rod 14 via the driving cylinder 15, and the screw rod 14 and the piston body 17 are advanced through a female screw of the nut-like screw body 16 by the rotation of the screw rod 14, to thereby feed the liquid cosmetic from the reservoir 11 to the application portion 30.

As illustrated in FIG. 1, an operating portion of the feeding member 13 is rotatably fitted into the rear end portion of the barrel body 12 and is exposed therefrom, the operating portion having a cylindrical shape closed by fitting a crown 13a into the rear end thereof. The driving cylinder 15 is fitted into the feeding member 13 and fixed in the rotating direction. The screw body 16 is mounted in the driving cylinder 15 so as to be fixed in the rotating direction and to be relatively movable in the axial direction. Reference numeral 13b denotes a spring member that rearwardly biases the feeding member 13 serving as a rotating body. In this applicator, a sealing portion 18, a joint member 19, a front barrel 20, and the application portion 30 are fitted into the front end portion 12a of the barrel body 12. The liquid cosmetic is stored in the reservoir 11 of the barrel body 12, and the liquid cosmetic fed out from the reservoir 11 is discharged to the application portion 30 through a flow path in the joint member 19 to be applicable. The applicator is configured such that, after use, a cap 40 can be attached to the front barrel 20 so as to cover the application portion 30 and the front barrel 20.

In FIG. 1, reference numeral 21 denotes a stirring ball configured to stir the liquid cosmetic in the reservoir 11 by reciprocating motion, and reference numeral 22 denotes a sealing ball. Reference numeral 41 denotes an inner cap in the cap 40, and reference numeral 42 denotes a spring for biasing the inner cap rearward. The stirring ball 21 may be omitted.

Reference numeral 23 denotes a stopper having a ring-shaped portion mounted between the rear end of the front barrel 20 and the front surface of a stepped portion of the front end portion 12a of the barrel body 12, in order to locate the sealing portion 18, the joint member 19, the front barrel 20, and the application portion 30 at a position for closing the flow path of the liquid cosmetic toward the application portion 30 when not in use. In the stopper 23, a part of a ring-shaped portion is cut off, and a knob piece is integrally formed on the opposite side of the cut-off portion. When the knob piece is pulled, the diameter of the ring-shaped portion is enlarged from the cut-off portion, to be removed from between the rear end of the front barrel 20 and the front end portion 12a of the barrel body 12.

As illustrated in FIG. 1, when not in use, the sealing ball 22 fits into and seals the inner diameter portion of the sealing portion 18 serving as a sealing ball receiver, and thus the liquid cosmetic does not flow toward the application portion 30. At the time of use, when a user pulls out the stopper 23 from the barrel body 12 and pushes the front barrel 20 to the rear end side, a small-diameter portion of the rear end of the joint member 19 abuts against the sealing ball 22, and the sealing ball 22 is removed from the inner diameter portion of the sealing portion 18 and enters into the reservoir 11. Then, the liquid cosmetic in the reservoir 11 flows into a liquid flow channel of the application portion 30 from an inner diameter portion of the joint member 19 and is supplied into the application portion 30 from its inside. Then, the liquid cosmetic is applicable to a target portion.

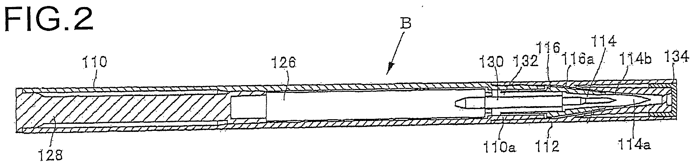

As illustrated in FIG. 2, a liquid cosmetic applicator B that is a type of applicator with built-in cosmetic has a barrel 110 including an inner cotton 126 impregnated with a cosmetic; an application portion 114 that is provided at a barrel tip 110a and is configured to apply the cosmetic to a target; and a holding member 116 that covers the outer periphery of the side of the barrel 110 (base side) of the application portion 114 except for a tip 114a of the application portion 114. The applicator with built-in cosmetic has a structure in which the cosmetic inside the barrel 110 is supplied to the application portion 114, and has a cap 112 removably attached to the barrel tip 110a to cover the application portion 114 and the holding member 116.

The tip 114a of the application portion 114 is sharpened. The application portion 114 has a brush-like shape in which fibers are bundled. Specifically, the application portion 114 is formed by arranging (sharpening) a plurality of resin-made fiber bundles (specific examples: the material of the fibers is made of polybutylene terephthalate (PBT) and the fiber thickness is 0.1 to 0. 15 mm) in a brush-like shape to thereby taper the tip 114a, and integrating the rear end portion by heat welding and expanding the diameter in a flange-like shape. Alternatively, the application portion 114 may be formed by solidifying other fibers.

As illustrated in FIG. 2, the applicator with built-in cosmetic of this embodiment includes the inner cotton 126 of an ink absorber from the central portion to the tip 110a of the tip portion in the barrel 110, and the inner cotton 126 is sealed and supported by a tail plug 128 inserted from the rear end of the barrel 110.

An ink relay core 130 made of an open-cell foam is disposed in the opening of the barrel tip 110a. The tail end of the relay core 130 is fitted into the tip of the inner cotton 126, while the tip thereof is fitted into the tail end portion of the application portion 114, thereby guiding the ink occluded in the inner cotton 126 to the application portion 114. The relay core 130 is mounted via a substantially cylindrical support 132 in the barrel tip 110a that is stepped relative to the main body and has a slightly reduced diameter (reduced diameter by the thickness of the cap 112). The cylindrical rear end portion of the holding member 116 is fitted between the outer periphery of the support 132 and the inner periphery of the barrel tip 110a.

The tip of the holding member 116 is located forward of the barrel tip 110a and covers the outer peripheral surface 114b of the application portion 114, and the holding member outer peripheral surface 116a is tapered in a shape of a conical side surface or a tapered shape.

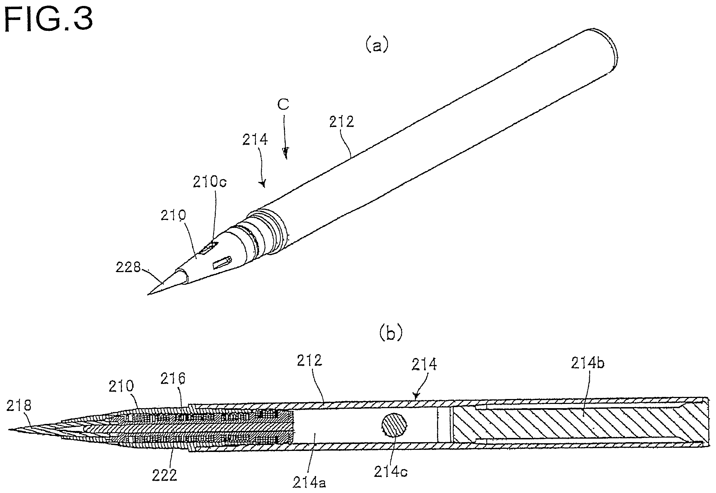

As illustrated in FIG. 3(a), the liquid cosmetic applicator C that is a collector-type applicator has a barrel 214 in which a front barrel 210 and a barrel body 212 at the rear side of the front barrel 210 are fitted into each other. As illustrated in FIG. 3(b), a collector 216, which is formed in a comb-like shape in an embodiment in which a plurality of sheet portions are arranged in the axial direction, is disposed in the front portion of the barrel 214, and a coating liquid is stored in a storage space 214a in the rear portion in the barrel 214.

The barrel body 212 at the rear portion of the barrel 214 is formed in a pipe shape that communicates with the inside and is opened in the front and rear direction. A tail plug 214b is fitted to a rear portion of the barrel body 212 that is also a rear portion of the barrel to close the rear portion of the barrel body 212. A space in the barrel 214 (also a space in the barrel body 212) sandwiched between a front end of the tail plug 214b and a rear end of the collector 216 is the storage space 214a.

In the storage space 214a, an impregnation body such as an inner cotton is not disposed, but a coating liquid is directly stored, and a stirring body (e.g., ball) 214c configured to stir the coating liquid is disposed.

The front barrel 210, the barrel body 212, the collector 216, and the cap may be resin molded products. A ball material made of a metal or a resin can be used for the stirring body 214c.

The collector 216 is covered and held by the front barrel 210 and the barrel body 212.

A writing portion 218 formed of a brush body having a tapered shape protrudes from an opening at the front end portion of the front barrel 210, and a cap covering the writing portion 218 is removably fitted to the front barrel 210 . The front barrel 210 has a substantially conical side surface shape and is formed to be tapered, and the front barrel 210 is desirably formed such that the tip angle thereof is substantially equal to the tip angle of the writing portion 218.

The writing portion 218 is a tapered brush body made of resin fibers, natural fiber bundles, or a resin porous body. The rear end portion of the writing portion 218 has an enlarged diameter in a flange shape. This enlarged portion is engaged with the inside of the front barrel 210 and prevents the front barrel 210 from being removed. The writing portion 218 is suitably a brush body, but any other application body configured to apply a coating liquid can be used.

In the cap, a cup-shaped inner cap configured to cover the writing portion 218 in order to enhance airtightness is disposed so as to be movable back and forth, and a spring for biasing the inner cap rearward is disposed.

A bellows-like collector 216 is disposed behind the writing portion 218 in the inside of the hollow tapered front barrel 210, and a core 222 penetrates through the hollow portion of the collector 216 and is disposed therein. The core 222 can be formed of a capillary member such as a resin fiber bundle, a natural fiber bundle, or a resin porous body. In the core 222, the core 222 does not protrude from the rear end portion of the collector 216 into the storage space 214a of the barrel 214 (see FIG. 3(b)). The rear end surface of the core 222 substantially matches the rear end surface of the collector 216. By matching the core 222, the rear end of the core 222 does not protrude into the storage space 214a, and the volume in the storage space 214a can be secured. Since the rear end of the core 222 does not protrude into the storage space 214a, when the stirring body 214c is provided in the storage space 214a, the stirring body 214c does not collide with the core 222 and does not deform the core 222 even if the stirring body 214a moves in the storage space 214a. Therefore, the coating liquid can sufficiently penetrate through the core. A difference between the higher and lower concentrations of the pigment depending on the porosity of the core will be described.

The liquid cosmetic applicator of the above embodiment has been described with reference to, for example, a liquid cosmetic applicator of liquid eyeliner or liquid eye shadow that is a cosmetic composition as the aqueous liquid cosmetic of the present disclosure. However, the present disclosure is not limited thereto, and the present disclosure can also be applied to an eyebrow applicator for drawing a line on eyebrows and for drawing a line on a skin.

The applicator of rotary extension type illustrated in FIG. 1 is used as a liquid pressing mechanism of the liquid cosmetic applicator of the above embodiment, but a liquid cosmetic applicator of knock extension type may be used.

The aqueous liquid cosmetic of the first disclosure having such a composition is a cosmetic stored in a pen-type applicator having a brush at an application portion. Since the aqueous liquid cosmetic contains a suitable combination of raw materials, for example, at least an iron oxide pigment, a polyorganic acid or a polyorganic acid salt, emulsion particles containing acrylate copolymer, a nonionic surfactant, and an aqueous solvent, even when an iron oxide pigment having a high specific gravity, such as red ocher, is used as a colorant of the liquid cosmetic, the iron oxide pigment is re-dispersed in the liquid cosmetic stored in the applicator by a stirring member. Therefore, sedimentation due to aggregation of the iron oxide pigment is prevented in the liquid cosmetic (after dispersion), or color separation during writing is prevented by adjustment of a core or a writing portion. Thus, the liquid cosmetic has excellent dispersibility and stability over time. Therefore, even when the cosmetic composition of the present disclosure is stored for a long period of time in a container such as a liquid cosmetic applicator to be used, the dispersibility and stability over time of the cosmetic are maintained in the container over a long period of time. Therefore, the resultant aqueous liquid cosmetic is suitable as a liquid cosmetic such as an eyeliner liquid that hardly causes color separation at the tip of a brush even in, for example, the eyeliner applicator container using the brush 30 as an application body as illustrated above in FIG. 1.

In addition, when the liquid is charged in the barrel at the time of production, stirring of the liquid is not required. In addition, the applicator container as illustrated in FIG. 1 or FIG. 3 is provided with a stirring body. However, a user needs not to perform stirring, and no color change occurs at the time of application.

### Aqueous Liquid Cosmetic of Second Disclosure

The aqueous liquid cosmetic of the second disclosure is a cosmetic stored in a type of applicator in which liquid is fed from an application liquid storage portion to an application portion using capillary force, and the cosmetic contains at least a pigment containing carbon as a main component, a water-soluble acrylic copolymer selected from Group X below, core-shell-type emulsion particles containing acrylate copolymer, and an aqueous solvent.

Group X: styrene acrylate copolymer, styrene acrylic acid copolymer, and methylstyrene-acrylic acid copolymer.

Examples of the pigment containing carbon as a main component (carbon-based black pigment) used in the second disclosure include at least one (alone or a mixture of two or more types, the same shall apply hereinafter) of carbon black, graphite, or spherical graphite. One selected from carbon black and/or graphite is preferred, and carbon black is particularly preferred.

The particle shape of the pigment containing carbon as a main component is not particularly limited and may be any shape such as a spherical, granular, rod-like, needle-like, plate-like, or amorphous shape. The average particle size of the pigment (particles) containing carbon as a main component that can be used before dispersion (at the time of blending) varies depending on the usage of the cosmetic and the type of pigment. For example, carbon black having an average particle size of 2000 nm or less, preferably 100 to 1000 nm, can be used.

In the present disclosure (including Examples), the "average particle size" refers to a value measured and calculated by a dynamic light scattering method [particle size analyzer FPAR-1000, available from Otsuka Electronics Co., Ltd.].

The (total) content of the pigment containing carbon as a main component is preferably 1 to 20 mass%, and more preferably 4 to 10 mass%, relative to the entire amount of the cosmetic composition.

When the content of the pigment containing carbon as a main component is less than 1 mass%, the color development is insufficient and the concealing ability is also insufficient. Meanwhile, when the content exceeds 20 mass%, the viscosity is increased and writing is hindered, which is not preferred.

Examples of the water-soluble acrylic copolymer used in the second disclosure include at least one selected from Group A: (styrene/acrylate) copolymer and ammonium acrylate copolymer. Examples of the (styrene/acrylate) copolymer include styrene-alkyl acrylate copolymers. Examples of commercially available products of the polymer include SYNTRAN EX149PE (available from INTERPOLYMER). Examples of the ammonium acrylate copolymer include an alkyl acrylate copolymer. Examples of commercially available products of this polymer include SYNTRAN 5402 (available from INTERPOLYMER).

The content of such a water-soluble acrylic copolymer is 0.1 to 5.0 mass%, preferably 0.2 to 3.0 mass%, and more preferably 0.5 to 2.0 mass%, relative to the entire amount of the liquid cosmetic.

When the content of the water-soluble acrylic copolymer is 0.1 mass% or more, dispersibility and dispersion stability of the pigment can be improved. Meanwhile, when the content thereof is 5.0 mass% or less, an increase in the viscosity of the liquid cosmetic can be prevented, and good application performance can be achieved.

In the second disclosure, the core-shell-type emulsion particles containing the acrylate copolymer may be a random copolymer, a graft copolymer, a block copolymer, or a core-shell-type copolymer. In particular, an acrylic resin emulsion or a urethane-acrylic composite resin emulsion is preferred, wherein the emulsion contains particles having a core-shell structure in which the core is an acrylic resin such as poly(meth)acrylate or a urethane resin such as polyurethane and the shell is an acrylic resin such as poly (meth) acrylate or a urethane resin such as polyurethane.

Specific examples thereof include EMUPOLY-CE-119N (available from Gifu Shellac Mfg. Co., Ltd.) which is an emulsion of core-shell-type copolymer of ethylhexyl acrylate/methacrylic acid copolymer [(acrylate/methylstyrene/styrene) copolymer ammonium].

From the viewpoint of film-forming properties and fixation of drawn lines, it is preferable to contain acrylate copolymer, and more preferably, it is desirable to use the core-shell-type polymer emulsion described above.

In these emulsion particles, a resin component is usually finely dispersed in an aqueous component at a concentration of 20 to 60 mass% as a solid content. The blended amount thereof is 1 to 40 mass%, preferably 3 to 20 mass%, and more preferably 5 to 15 mass% in view of solid content concentration relative to the entire amount of the liquid cosmetic.

When the content of the emulsion particles is less than 1 mass%, the fixation and the water-resistant fixation are lowered. Meanwhile, when the content thereof is more than 40 mass%, the viscosity of the liquid cosmetic is increased, leading to difficulty in application of the cosmetic.

The aqueous solvent used in the second disclosure is used as a solvent of a liquid cosmetic. Examples thereof include at least one (alone or a mixture of two or more types, the same shall apply hereinafter) of water, a lower alcohol having 6 or less carbons, or a polyhydric alcohol. Specific examples of the lower alcohol having 6 or less carbons include at least one of methyl alcohol (methanol), ethyl alcohol (ethanol), n-propylalcohol, isopropyl alcohol, n-butyl alcohol, sec-butyl alcohol, tert-butyl alcohol, isobutyl alcohol, pentyl alcohol, ethylene glycol, or propylene glycol.

Examples of the polyhydric alcohol include glycerin, erythritol, threitol, arabinitol, xylitol, ribitol, propylene glycol, 1,3-butylene glycol, 1,2-pentanediol, ethylene glycol, diethylene glycol, and pentaerythritol.

In particular, it is desired to use ethyl alcohol (ethanol), from the perspective of safety and handleability.

The total content of the aqueous solvent to be used is preferably 45 to 85 mass%, and more preferably 50 to 80 mass%, relative to the entire amount of the liquid cosmetic, in view of stability and dispersion stability of the pigment containing carbon as a main component, and particularly in view of stability at low temperature.

When the content of the aqueous solvent is 45 mass% or more, smooth writing and moisture retaining properties can be achieved, and an antiseptic effect can be slightly achieved. Meanwhile, when the content thereof is 85 mass% or less, the bleeding resistance of a drawn line is further improved.

Water serving as a solvent used in the second disclosure can be distilled water, ion-exchanged water, purified water, pure water, or ultrapure water. In view of further improving stability over time and dispersibility of the carbon black, the ratio of water to the aqueous solvent (water + a solvent other than water) is preferably 50 to 100 mass% (0.5 to 1.0), and particularly preferably 80 to 95 mass%.

Further, the aqueous liquid cosmetic of the second disclosure may contain, in addition to the above-described components, an optional component used in ordinary liquid cosmetics. Specifically, the aqueous liquid cosmetic may contain a predetermined amount of a preservative, an antioxidant, a neutralizing agent, an ultraviolet absorber, a chelating agent, a humectant such as 1,3-butylene glycol, a beauty ingredient, a perfume, a viscosity/stickiness modifier, or another dispersant such as polyethylene glycol alkyl ether as long as the effects of the present disclosure are not impaired. Besides the inorganic pigment other than the pigment containing carbon as a main component, an additional colorant such as an organic pigment, a water-soluble dye, or a resin particle pigment may optionally be contained, as long as the effects of the present disclosure are not impaired.

The aqueous liquid cosmetic of the second disclosure preferably has a viscosity of 15 mPa·s or less, preferably 10 mPa·s or less, and more preferably 2 to 8 mPa·s.

When the viscosity exceeds 15 mPa·s, the flowability of the aqueous liquid cosmetic is significantly decreased, which is not preferred. Adjustment of the above viscosity range can be performed, for example, by suitably combining the respective components such as the pigment containing carbon as a main component to be used and its average particle size, the water-soluble acrylic copolymer, the emulsion particles containing acrylate copolymer, and the aqueous solvent, by combining the contents of the respective components within a suitable range, or by a suitable dispersion method.

In the present disclosure, regarding the viscosity measurement conditions (including Examples described below), specifically, the resultant cosmetic composition is subjected to measurement with an ELD-type viscometer available from Toki Sangyo Co., Ltd using a standard rotor: 50 rpm (shear rate: 192 [s-1]) at a temperature of 25°C.

Furthermore, the aqueous liquid cosmetic of the second disclosure has a surface tension at a temperature of 25°C preferably in a range of 35 to 60 mN/m, more preferably in a range of 35 to 45 mN/m as measured by the Wilhelmy method (plate method), from the viewpoint of good spreadability during application. When the surface tension is 35 mN/m or more, smooth application can be performed without bleeding or unevenness in applied lines, and dripping (dropping) is reduced even in the case where the cosmetic has a low viscosity. Meanwhile, when the surface tension is 60 mN/m or less, followability of the application liquid is improved and smooth application can be performed.

In the present disclosure (including Examples described below), the measured surface tension refers to a value obtained at a temperature of 25°C using a CBVP-Z type surface tensiometer (plate method) available from Kyowa Interface Science Co., Ltd.

The surface tension can be adjusted to fall within the above range by suitably combining the amounts of the respective components such as a pigment containing carbon as a main component, carbon black, a water-soluble acrylic copolymer, and emulsion particles containing acrylate copolymer.

When the aqueous liquid cosmetic of the second disclosure is prepared by using the above-mentioned pigment containing carbon as a main component, such as carbon black, water-soluble acrylic copolymer, emulsion particles containing acrylate copolymer, aqueous solvent, and additional component under suitable dispersion conditions using a dispersing machine such as a homomixer, a sand mill, an ultrasonic homogenizer, or a high-pressure homogenizer, the average particle size of carbon black in the cosmetic (after dispersion) can be adjusted to 200 nm or less.

Preferably, when a (multiple type) ultrasonic homogenizer or a high-pressure homogenizer is used as the dispersion machine, the average particle size of the pigment containing carbon as a main component in the cosmetic (after dispersion) can be adjusted to 200 nm or less by setting the dispersion conditions; for example, the frequency is set to 20 to 30 KHz in the case of an ultrasonic homogenizer, or the pressure is set to 150 to 245 MPa in the case of a high-pressure homogenizer.

The resultant aqueous liquid cosmetic can be stored in a type of applicator in which liquid is fed from an application liquid storage portion to an application portion using capillary force. For example, the aqueous liquid cosmetic can be stored (charged) in a pen-type liquid cosmetic applicator (container) having a brush or a pen core as an application portion for use.

The liquid cosmetic applicator that can be used is not particularly limited as long as it is a liquid cosmetic applicator equipped with a brush or a pen core for, for example, an eyeliner or an eyebrow pen. Preferred examples of the liquid cosmetic applicator include an applicator having, as application means, a brush (brush pen) or a pen core for an eyeliner or an eyebrow pen, or an application body formed of rubber, an elastomer, or a closed-cell foam having restorability, and having a container for charging the liquid cosmetic.

The aqueous liquid cosmetic of the second disclosure is stored in a liquid cosmetic applicator having an application portion provided with a brush or a pen core for use.

In the present disclosure, a suitable liquid cosmetic applicator is not particularly limited as long as it is an eyeliner, an eyeshadow pen, an eyebrow pen suitable for point makeup around eyes, or a liquid cosmetic applicator having a structure capable of suitably applying a liquid cosmetic composition (application liquid) to a skin. Preferred examples of the liquid cosmetic applicator include an liquid cosmetic applicator including an inner cotton-type container configured to hold a liquid cosmetic in an inner cotton or a collector-type container configured to hold a liquid cosmetic in a sheet body, and a liquid cosmetic applicator having an application liquid storage portion that directly stores a liquid cosmetic composition and a knock-type or rotary (pivotal) discharging mechanism of the application liquid, which are excellent in usability, convenience, and application properties.

In the second disclosure, a preferred liquid cosmetic applicator is a liquid cosmetic applicator including at least an application portion and an application liquid storage portion, wherein the application portion is formed of a brush, the brush includes fibers having a cross section with a shape other than a circular shape, and the liquid cosmetic composition of the present disclosure is stored in the application liquid storage portion.

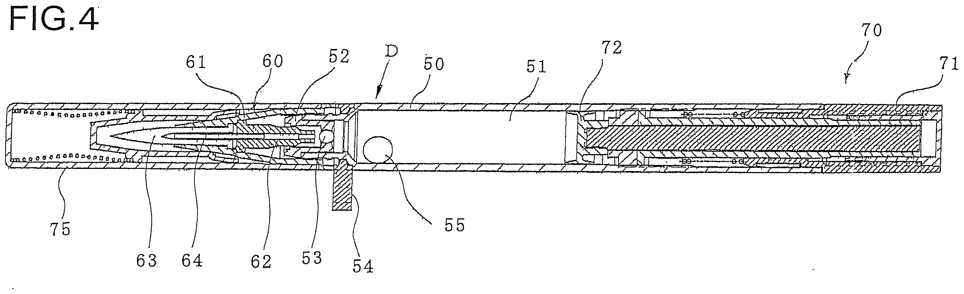

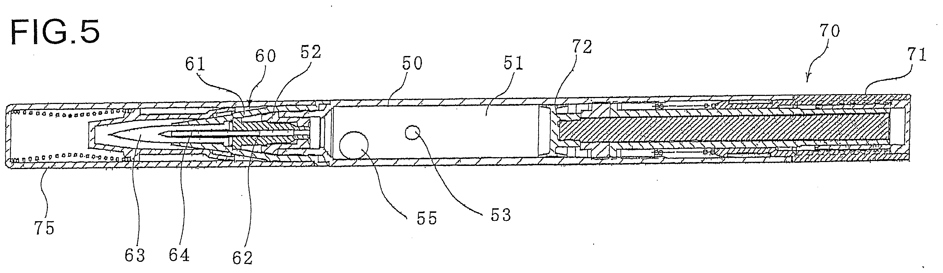

Examples of the liquid cosmetic applicator of this embodiment include a liquid cosmetic applicator illustrated in FIGS. 4 and 5.

As illustrated in FIG. 4, the liquid cosmetic applicator D according to the embodiment of the second disclosure includes a reservoir (storage portion) 51 of an application liquid that is the liquid cosmetic composition of the present disclosure, which has the above structure and is disposed inside of the front of the barrel body 50 serving as the applicator body; a plug body 52 mounted on the front end portion of the barrel body 50 and is configured to seal the reservoir (storage portion) 51 before start of use; and a sealing ball 53. The sealing ball 53 is mounted to the plug body 52, and maintains the sealing condition of the reservoir (storage portion) 51. When the sealing ball 53 is removed from the plug body 52, the sealing condition of the reservoir (storage portion) 51 is released, to thereby allow the application liquid to be discharged from the reservoir (storage portion) 51.

The liquid cosmetic applicator D also includes a stopper 54 removably mounted on the outer peripheral surface of the front end portion of the barrel body 50; and a front barrel 60 that moves in the axial direction by the length of the stopper 54 and is directly joined to the barrel body 50 when the stopper 54 is removed at the start of use.

The front barrel 60 includes a front barrel body 61; a pipe joint 62 that is mounted on the front barrel body 61 and is capable of supplying the application liquid from the reservoir 51 to the brush (application portion) 63; and an application liquid conducting tube 64 connecting the pipe joint 62 and the brush (application portion) 63.

With this configuration, in the state illustrated in FIG. 4 (the state before the start of use), the stopper 54 is attached to the barrel body 50, and thus the front barrel 60 does not move, and the sealing condition of the reservoir (storage portion) 51 is maintained by the plug body 52 (sealing ball 53) that seals the reservoir (storage portion) 51. As illustrated in FIG. 5, when the stopper 54 is removed at the start of use, the front barrel 60 moves in the axial direction by the length of the stopper 54 and abuts against the barrel body 50. That is, the pipe joint 62 of the front barrel 60 and the plug body 52 are joined to each other, and the sealing ball 53 attached to the plug body 52 is removed by the tip of the pipe joint 62.

As a result, the sealing state of the reservoir (storage portion) 51 is released, and the application liquid can be supplied from the reservoir 51 to the brush (application portion) 63.

An application liquid discharging mechanism 70 is provided at the rear end portion of the barrel body 50. The application liquid discharging mechanism 70 is configured to allow a piston 72 to move forward by a predetermined distance by rotating a canopy 71 and to discharge a predetermined amount of the application liquid. The brush 63 is covered with a cap 75.

In FIGS. 4 and 5, reference numeral 55 denotes a stirring ball configured to stir the application liquid. The application liquid discharging mechanism 70 is disclosed in JP 2012-157611 A, which has been proposed by the present applicant. The application liquid discharging mechanism 70 is not limited to a rotary type in which the application liquid is discharged by rotating the canopy 71, but may be a knock type in which the application liquid is discharged by pressing the canopy 71.

In the liquid cosmetic applicator D of the second disclosure configured as described above, firstly, the stopper 54 is removed, and the cap 75 (front barrel body 61) is pushed (moved) to the rear side of the barrel body 50. As a result, the end portion of the pipe joint 62 abuts against the sealing ball 53, the sealing ball 53 is removed from the plug body 52, and the application liquid can be supplied. Thereafter, the cap 75 is removed, the canopy 71 is rotated, and the piston 72 is allowed to move forward by a predetermined distance. Thus, a predetermined amount of the application liquid is discharged to the brush 63, and the application liquid is applied to an application site of the user.

The shape, structure, and material of the brush 63 to be used are not particularly limited as long as the brush 63 can be suitably applied to an eyeliner, an eyeshadow, an eyebrow, or a skin. In the embodiment of the second disclosure, the brush is formed of an aggregate of fibers (brush hairs) made of linear synthetic fibers with tapered tips and having a non-circular deformed cross-section, and is composed of a plurality of types of brush hairs having different cross-sectional shapes. The brush hairs having a deformed cross-section refer to brush hairs having a shape other than a circular shape in a plane perpendicular to the axial direction of the brush hairs. Examples of the brush hairs include those having a cross-sectional shape such as a substantially star shape, a substantially cocoon shape, a substantially cross shape, a substantially triangular shape, a substantially Y-shape, a substantially semicircular shape, or a substantially rectangular shape, and those having a shape provided with at least one or more projections. Examples of the synthetic fibers include synthetic resin fibers produced by melt-spinning of a thermoplastic polymer, for example, a polyester such as polyethylene terephthalate, polybutylene terephthalate, or a polyethylene terephthalate-isophthalate co-condensation polymer; a polyolefin such as polyethylene or polypropylene; or a polyamide such as nylon 6, nylon 610, or nylon 612.

In the liquid cosmetic applicator D of the second disclosure configured as described above, when multiple types of brush hairs having different deformed cross sections are used in the brush serving as the application portion, a decrease in number of voids (gaps) between the brush hairs is suppressed, and strong elasticity can be imparted to the brush. In addition, the above aqueous liquid cosmetic of the second disclosure does not blur on a skin even when it is applied on a skin, and contains a pigment containing carbon as a main component, which causes less dripping (dropping) and has a low viscosity. Therefore, the properties of drawing darkly can be achieved. Then, the aqueous liquid cosmetic serving as the application liquid can be sufficiently contained in the brush due to the synergistic effect of the characteristics of the aqueous liquid cosmetic composition and the structural characteristics of the liquid cosmetic composition applicator, and the aqueous liquid cosmetic can be applied comfortably. Moreover, in the present disclosure, the aqueous liquid cosmetic contains, as a colorant, a pigment such as carbon black dispersed by resin components, and thus can be claimed as a composition containing no surfactant (free of a surfactant) . Therefore, both fixation and cleansing properties of drawn lines can be highly achieved, and the resultant aqueous liquid cosmetic is suitable for a liquid cosmetic applicator of a makeup cosmetic such as an eyeshadow, an eyeliner, an eyebrow cosmetic, or a mascara.

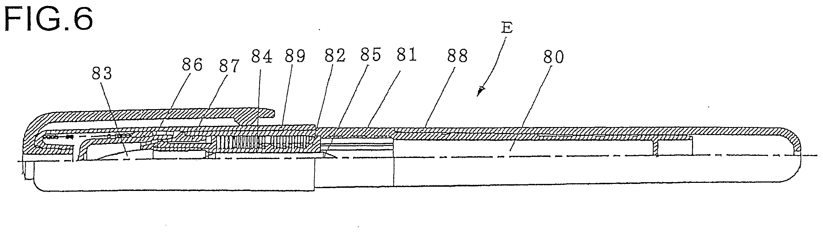

FIG. 6 illustrates a direct-liquid-type liquid cosmetic applicator of a collector type, which stores the liquid cosmetic composition of the present disclosure.

As illustrated in FIG. 6, the liquid cosmetic applicator E is, for example, an applicator including a tank portion 81 serving as a barrel that directly stores the liquid cosmetic composition 80 of the second disclosure without absorption of the liquid cosmetic composition 80 in an inner cotton or a like material. The front portion of the tank portion 81 includes a sheet body (ink holding body, collector member) 82 configured to temporarily hold the liquid cosmetic 80 pushed from the tank portion 81 when air in the tank portion 81 expands due to temperature rise, in order to prevent dropping of the liquid cosmetic 80 from a pen tip or an air pore, and the tip of the collector member 82 is provided with a brush-type pen tip (brush) 83 serving as an application body. The brush 63 having a deformed cross section used in the above liquid cosmetic applicator D can be used as the brush-type pen tip (brush) 83.

Discharging the liquid cosmetic from the tank portion 81 to the pen tip 83 is performed by discharging the liquid cosmetic 80 from the tank portion 81 to the pen tip 83 serving as the application portion via a relay core 85 having an application liquid flow channel 84 provided in a central hole of the collector member 82.

In FIG. 6, reference numerals 86 and 87 denote holder members, reference numeral 88 denotes a rear barrel fixed on the rear portion of the tank portion 81, and reference numeral 89 denotes a cap having an inner cap. For discharge of the liquid cosmetic, the rear portion of the pen tip 83 may be disposed directly in the tank portion 81 without interposing the relay core 85.

The liquid cosmetic applicator E of this embodiment can be used in the same manner as the liquid cosmetic applicator D by removing the cap 89.

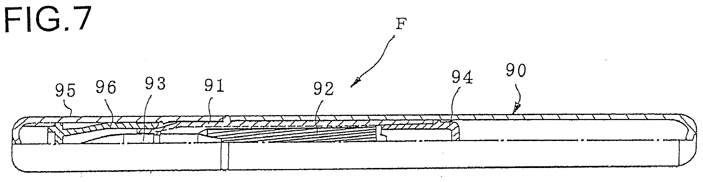

FIG. 7 illustrates a case where the aqueous liquid cosmetic of the present disclosure is used in an inner cotton-type liquid cosmetic applicator.

As illustrated in FIG. 7, the liquid cosmetic applicator F of this embodiment has, for example, a structure in which a cosmetic applicator body 90 includes an inner barrel 91; the inner barrel 91 accommodates an impregnation body 92 formed of, for example, an inner cotton impregnated with the liquid cosmetic composition of the second disclosure; the tip end of the impregnation body 92 is provided with an application body 93 formed of a brush having a deformed cross section used in the liquid cosmetic applicator D for application of the liquid cosmetic; and a tail plug 94 is fixed at the rear end of the inner barrel 91. Reference numeral 95 denotes a cap body having an inner cap portion 96. The liquid cosmetic applicator F of this embodiment can be used by removing the cap 95.

The liquid cosmetic applicator illustrated in FIG. 6 or FIG. 7 is configured as described above, and thus the liquid cosmetic applicator achieves sufficient impregnation of the brush with the aqueous liquid cosmetic of the second disclosure serving as the application liquid and enables comfortable application.

In the liquid cosmetic applicators D to F according to the embodiments of the second disclosure described above, the fiber of the brush used has a non-circular deformed cross section. As described above, the aqueous liquid cosmetic according to the second disclosure has such a low density that it does not blur on a skin even when it is applied onto a skin, causes less dripping (dropping), and enables dense drawing. Therefore, the brush used in each of the embodiments A to F may be formed of liner synthetic fibers having a tapered tip and may have a circular cross section.

The liquid cosmetic applicator of the above embodiment of the second disclosure has been described with reference to, for example, a liquid cosmetic applicator of liquid eyeliner or liquid eye shadow that is a cosmetic composition as the aqueous liquid cosmetic of the second disclosure. However, the present disclosure is not limited thereto, and the present disclosure can also be applied to an eyebrow applicator for drawing a line on eyebrows and for drawing a line on a skin. The applicator of rotary extension type illustrated in FIG. 4 is used as a liquid pressing mechanism of the liquid cosmetic applicator of the above embodiment, but a liquid cosmetic applicator of knock extension type may be used.

The aqueous liquid cosmetic of the second disclosure having such a composition is a cosmetic stored in a type of applicator in which liquid is fed from an application liquid storage portion to an application portion using capillary force. Since the aqueous liquid cosmetic contains a suitable combination of raw materialssuch as at least a pigment containing carbon such as carbon black as a main component a water-soluble acrylic copolymer, emulsion particles containing acrylate copolymer, and an aqueous solvent, makeup durability can be achieved even when the cosmetic contains a pigment containing carbon as a main component and does not contain a surfactant. Therefore, the thus-provided aqueous liquid cosmetic can highly achieve both fixation and cleansing properties of drawn lines, and is suitable as a makeup cosmetic such as an eyeshadow, an eyeliner, an eyebrow cosmetic, or a mascara.

### Examples

The present disclosure will next be described in further detail with reference to Examples and Comparative Examples, but the present disclosure is not limited to the following Examples.

First Disclosure: Examples 1 to 12 and Comparative Examples 1 to 4 Cosmetic compositions as aqueous liquid cosmetics of the first disclosure having formulations shown in Table 1 below (blending unit: mass%, total amount 100 mass%) were prepared by the following method. The viscosity and pH of each of the liquid cosmetics were measured by the above-described measurement methods, and the liquid cosmetic was evaluated for color development property, fixation, sebum resistance, water-resistant fixation, and drying property by the following evaluation methods.

The results are shown in Table 1 below. It was confirmed that the average particle size of the pigment (colorant) containing the iron oxide pigment in each of the aqueous liquid cosmetics (after dispersion) of Examples 1 to 12 and Comparative Examples 1 to 4 was 300 nm or less.

### Method for Preparing Aqueous Liquid Cosmetic

A cosmetic composition as an aqueous liquid cosmetic having the formulation shown below in Table 1 was prepared by a typically used procedure. Specifically, an iron oxide pigment as a colorant and a polyorganic acid as a dispersant were added to purified water as a vehicle and were dispersed using LABSTAR (available from Ashizawa Finetech Ltd.) as a dispersing machine. Then, other components were added to the dispersion, followed by mixing to prepare a cosmetic composition.

### Method of Measuring pH

The pH of each of the prepared aqueous liquid cosmetics was measured at 25°C with a glass electrode pH meter.

### Method for Measuring Viscosity

The viscosity of each of the aqueous liquid cosmetics prepared by the above-described method was measured using an ELD-type viscometer available from Toki Sangyo Co., Ltd with a standard rotor: 50 rpm (shear rate: 192 [s-1]) at 25°C.

### Method of Evaluating Color Development Property

The prepared aqueous liquid cosmetic was charged in the direct-liquid-type eyeliner of FIG. 1 and was applied to a skin. The color development property was evaluated according to the following evaluation criteria.

### Evaluation criteria:

A: Very good color development.
B: Good color development.
C: Slightly poor color development.
D: Poor color development.

### Method of Evaluating Fixation

The prepared aqueous liquid cosmetic was charged in the direct-liquid-type eyeliner of FIG. 1 and was applied to a skin. Then, the applied surface was rubbed with a ball of a finger to evaluate fixation according to the following evaluation criteria.

### Evaluation criteria:

A: No change in the drawn line by rubbing with a ball of a finger.
B: A slight change is observed in the drawn line by rubbing with a ball of a finger.
C: A change is observed in the drawn line by rubbing with a ball of a finger.

### Method of Evaluating Sebum Resistance

The prepared aqueous liquid cosmetic was charged in the direct-liquid-type eyeliner of FIG. 1 and was applied to a skin. Then, artificial sebum was applied and the applied surface was rubbed with a ball of a finger to evaluate sebum resistance according to the following evaluation criteria.

### Evaluation criteria:

A: No change in the drawn line by rubbing with a ball of a finger.
B: A slight change is observed in the drawn line by rubbing with a ball of a finger.
C: A change is observed in the drawn line by rubbing with a ball of a finger.

### Method of Evaluating Water-Resistant Fixation

The prepared aqueous liquid cosmetic was charged in the direct-liquid-type eyeliner of FIG. 1 and was applied to a skin. Then, the applied surface was rinsed with running water and rubbed with a ball of a finger to evaluate water-resistant fixation according to the following evaluation criteria.

### Evaluation criteria:

A: No change in the drawn line by rubbing with a ball of a finger.
B: A slight change is observed in the drawn line by rubbing with a ball of a finger.
C: A change is observed in the drawn line by rubbing with a ball of a finger.
D: The drawn line is erased by rubbing with a ball of a finger.

### Method of Evaluating Drying Property

The prepared aqueous liquid cosmetic was charged in the direct-liquid-type eyeliner of FIG. 1 and was applied to a skin. After the elapse of a certain period of time, a facial tissue was pressed to the drawn line, to evaluate drying property according to the following evaluation criteria.

### Evaluation criteria:

A: No liquid adheres to the facial tissue.
B: Liquid slightly adheres to the facial tissue.
C: Liquid adheres to the facial tissue.
D: Most liquid oozes and transfers to the facial tissue.

**[Table 1]**

| (Total amount 100 mass%) | | | | | | | | | | | | | | | | | |
|---|---|---|---|---|---|---|---|---|---|---|---|---|---|---|---|---|---|
| | | Example | | | | | | | | | | | | Comparative Example | | | |
| | | 1 | 2 | 3 | 4 | 5 | 6 | 7 | 8 | 9 | 10 | 11 | 12 | 1 | 2 | 3 | 4 |
| Colorant | Iron oxide *1 | 2.71 | 0.43 | 15.25 | 13.98 | 13.22 | 13.22 | 8.20 | 5.36 | 15.25 | 13.22 | 8.20 | 3.46 | 15.25 | 13.22 | 13.22 | |
| | Titanium oxide *2 | 11.55 | 3.72 | | | | | 10.64 | 6.25 | | | 10.64 | 11.31 | | | | |
| | Akaninum hydroxide *3 | 0.61 | 0.20 | | | | | 0.56 | 0.33 | | | 0.56 | 0.54 | | | | |
| | Carbon black *4 | 0.73 | 0.78 | | | | | | | | | | | | | | |
| | Red 226 | | 2.49 | | | | | | | | | | | | | | |
| | Blue No. 1 | | | | | | | | | | | | | | | | 13.22 |
| Envision resin (particles) | Ethylhexyl acrylate/methyl methacrylate copolymer [(acrylate/methylstyrene/ styrene) copolymer ammonim] *5 | 14.10 | 14.10 | 10.72 | 13.87 | 11.75 | 13.87 | 7.05 | 14.10 | 10.72 | 11.75 | 7.05 | 7.05 | | 11.75 | 11.75 | 11.75 |
| Polyorganic acid (salt) | Sodium polyaspartate *6 | 1.66 | 0.43 | 0.82 | 0.75 | 0.76 | 0.76 | 1.85 | 0.65 | 0.82 | 0.77 | 1.85 | 1.70 | 0.82 | | 0.77 | 0.77 |
| Nonionic surfactant | POE (20) cetyl ether | 0.05 | 0.09 | 0.30 | 0.28 | 0.30 | 0.30 | 0.17 | 0.14 | 0.30 | 0.31 | 0.17 | 0.09 | 0.30 | 0.31 | | 0.31 |
| | POE (30) behenyl ether | 0.20 | 0.21 | | | | | | | | | | | | | | |
| Added component | Citric acid | 0.03 | | 0.03 | 0.03 | 0.05 | 0.05 | 0.03 | 0.03 | | | | | | | | |
| | AMP | 0.00 | 0.04 | | | | | | | | | | | | | | |
| | Sodium dehydroacetate | 0.01 | 0.01 | | | | | | | | | | | 0.09 | | | |
| | Di(C12-15)pareth-10 phosphate '7 | | 0.50 | | | | | | | | | | | | | | |
| | EDTA-2Na | 0.11 | 0.11 | 0.06 | 0.06 | 0.06 | 0.06 | 0.04 | 0.02 | | | | | 0.06 | | | |
| | Acrylates copolymer *8 | | | | | | | | | | | | | 12.60 | | | |
| | Xanthan gum | | 0.01 | | | | | | 0.02 | | | | | | | | |
| Preservative | Phenoxyethanol | 0.50 | 0.51 | 0.47 | 0.47 | 0.50 | 0.50 | 0.50 | 0.50 | 0.47 | 0.51 | 0.50 | 0.50 | 0.47 | 0.51 | 0.51 | 0.51 |
| | Methylparaben | 0.20 | 0.19 | 0.15 | 0.14 | 0.14 | 0.14 | 0.19 | 0.15 | 0.15 | 0.14 | 0.13 | 0.16 | 0.15 | 0.14 | 0.14 | 0.14 |
| | Ethylparaben | 0.13 | 0.13 | 0.10 | 0.10 | 0.09 | 0.10 | 0.11 | 0.10 | 0.07 | 0.07 | 0.09 | 0.08 | 0.07 | 0.07 | 0.07 | 0.07 |
| | 1,2-Hexanediol | 1.00 | 1.00 | | | | | | | | | | 1.00 | | | | |
| | Ethylhexylglycerin | | | 0.15 | 0.15 | 0.11 | 0.11 | 0.18 | 0.17 | 0.15 | 0.11 | 0.18 | 0.13 | 0.15 | 0.11 | 0.11 | 0.11 |
| Aqueous solvent | Ethanol | | | | | | | | | | | | | 1.20 | | | |
| | 1,3-Butylene glycol | 10.48 | 10.08 | 9.81 | 8.36 | 9.08 | 9.39 | 9.96 | 9.58 | 9.90 | 9.25 | 9.40 | 8.43 | 10.38 | 9.25 | 9.25 | 9.25 |
| | Water (purified water) | Balance | Balance | Balance | Balance | Balance | Balance | Balance | Balance | Balance | Balance | Balance | Balance | Balance | Balance | Balance | Balance |
| Physical properties | Viscosity mPa·s 50 rpm (shear rate 192 s⁻¹) | 6.8 | 5 | 5 | 5.2 | 5.3 | 5.4 | 5.2 | 6.4 | 5 | 5.6 | 5 | 4.9 | 3.9 | 5 | 5.1 | 3.5 |
| | pH | 7.4 | 7.5 | 7.7 | 7.5 | 7.6 | 7.6 | 7.6 | 7.5 | 7.6 | 7.5 | 7.7 | 7.8 | 8 | 7.3 | 7.8 | 8 |
| Evaluation | Color development property | B | B | B | B | B | B | B | B | B | B | B | B | B | C | C | B |
| | Fixation | A | A | B | A | B | A | B | A | B | B | B | B | C | B | B | B |
| | Sebum resistance | A | A | B | A | B | A | B | A | B | B | B | B | C | B | B | B |
| | Water-resistant fixation | A | A | B | A | B | A | B | A | B | B | B | B | C | B | B | C |
| | Drying property | A | A | B | A | B | A | B | A | B | B | B | B | C | B | B | B |

| | | | | | | | | | | | | | | | | | |
|---|---|---|---|---|---|---|---|---|---|---|---|---|---|---|---|---|---|
| *1 to *8 in Table 1 above are as described below. *1: TALOX ABL-412HP/R-516P, available from Titan Kogyo, Ltd. *2: CR-50, available from DAITO KASEI KOGYO CO., LTD. *3: Contained in CR-50 above, available from DAITO KASEI KOGYO CO., LTD. *4: DK BLACK No. 2, available from DAITO KASEI KOGYO CO., LTD. *5: EMUPOLY-CE-119N, available from Gifu Shellac Mfg. Co., Ltd. *6: AQUADEW SPA-30B, available from AJINOMOTO *7: DD-P10, available from Nikko Chemicals Co., Ltd. *8: YODOSOL GH34F, available from AkzoNobel | | | | | | | | | | | | | | | | | |

As is clear from the results in Table 1 above, the aqueous liquid cosmetics of Examples 1 to 12, which fall within the scope of the first disclosure, are excellent in color development property, fixation, sebum resistance, water-resistant fixation, and drying property, as compared with the aqueous liquid cosmetics of Comparative Examples 1 to 4, which fall outside the scope of the present disclosure.

Second Disclosure: Examples 13 to 18 and Comparative Examples 5 to 7 Cosmetic compositions as aqueous liquid cosmetics of the second disclosure having formulations shown in Table 2 below (blending unit: mass%, total amount 100 mass%) were prepared by the following method. The viscosity, pH, and surface tension of each of the liquid cosmetics were measured by the above-described measurement methods, and the liquid cosmetic was evaluated for fixation, sebum resistance, water-resistant fixation, drying property, and coating property over time by the following evaluation methods.

The results are shown in Table 2 below.

### Method for Preparing Aqueous Liquid Cosmetic

A cosmetic composition as an aqueous liquid cosmetic having the formulation shown below in Table 2 was prepared by a typically used procedure. Specifically, a pigment containing, as a main component, carbon that is a colorant and a water-soluble acrylic copolymer were added to purified water as a vehicle and were dispersed using LABSTAR (available from Ashizawa Finetech Ltd.) as a dispersing machine. Then, other components were added to the dispersion, followed by mixing to prepare a cosmetic composition.

### Method of Measuring pH

The pH of each of the prepared aqueous liquid cosmetics was measured at 25°C with a glass electrode pH meter.

### Method of Measuring Viscosity

The viscosity of each of the aqueous liquid cosmetics prepared by the above-described method was measured using an ELD-type viscometer available from Toki Sangyo Co., Ltd under with a standard rotor: 50 rpm (shear rate: 192 [s-1]) at 25°C.

### Method of Evaluating Fixation

The prepared aqueous liquid cosmetic was charged in the direct-liquid-type eyeliner of FIG. 4 and was applied to a skin. Then, the applied surface was rubbed with a ball of a finger to evaluate fixation according to the following evaluation criteria.

### Evaluation criteria:

A: No change in the drawn line by rubbing with a ball of a finger.
B: A slight change is observed in the drawn line by rubbing with a ball of a finger.
C: A change is observed in the drawn line by rubbing with a ball of a finger.
D: The drawn line is erased by rubbing with a ball of a finger.

### Method of Evaluating Sebum Resistance

The prepared aqueous liquid cosmetic was charged in the direct-liquid-type eyeliner of FIG. 4 and was applied to a skin. Then, artificial sebum was applied and the applied surface was rubbed with a ball of a finger to evaluate sebum resistance according to the following evaluation criteria.

### Evaluation criteria:

A: No change in the drawn line by rubbing with a ball of a finger.
B: Slight change is observed in the drawn line by rubbing with a ball of a finger.
C: A change is observed in the drawn line by rubbing with a ball of a finger.
D: The drawn line is erased by rubbing with a ball of a finger.

### Method of Evaluating Water-Resistant Fixation

The prepared aqueous liquid cosmetic was charged in the direct-liquid-type eyeliner of FIG. 4 and was applied to a skin, followed by rinsing with running water. Then, the applied surface was rubbed with a ball of a finger to evaluate water-resistant fixation according to the following evaluation criteria.

### Evaluation criteria:

A: No change in the drawn line by rubbing with a ball of a finger.
B: A slight change is observed in the drawn line by rubbing with a ball of a finger.
C: A change is observed in the drawn line by rubbing with a ball of a finger.
D: The drawn line is erased by rubbing with a ball of a finger.

### Method of Evaluating Drying Property

The prepared aqueous liquid cosmetic was charged in the direct-liquid-type eyeliner of FIG. 4 and was applied to a skin. After the elapse of a certain period of time, a facial tissue was pressed to the drawn line, to evaluate drying property according to the following evaluation criteria.

### Evaluation criteria:

A: No liquid adheres to the facial tissue.
B: Liquid slightly adheres to the facial tissue.
C: Liquid adheres to the facial tissue.
D: Most liquid transfers to the facial tissue.

### Method of Evaluating Liquid Retaining Property

The prepared aqueous liquid cosmetic was charged in the direct-liquid-type eyeliner of FIG. 4, and the eyeliner was placed at 50°C for one week with the brush tip facing downward. Then, the liquid retaining property of the collector type was evaluated according to the following evaluation criteria.

### Evaluation criteria:

B: No liquid leaks from the brush tip.
C: Liquid slightly leaks from the brush tip.

**[Table 2]**

| (Total amount 100 mass%) | | | | | | | | | | |
|---|---|---|---|---|---|---|---|---|---|---|
| | | Example | | | | | | Comparative Example | | |
| | | 13 | 14 | 15 | 16 | 17 | 18 | 5 | 6 | 7 |
| Pigment | Carbon black *1 | 6.67 | 6.67 | 5.33 | 6.67 | 6.67 | 8.00 | 8.00 | 5.33 | 8.00 |
| Emulsionresin (particles) | Ethylhexyl acrylate/methyl methacrylate copolymer, (acrylate/methylstyrene/styrene) copolymer ammonium *2 | 11.75 | 10.58 | 11.75 | 11.75 | 12.93 | 11.75 | | | 10.58 |
| | Acrylate copolymer *3 | | | | | | | 9.00 | 12.60 | |
| Water-soluble resin (pigment dispersant) | (Styrene/acrylate) copolymer *4 | 2.00 | 2.00 | 1.20 | 2.00 | 2.00 | 2.40 | | | |
| | (Acrylates/octylacrylamide) copolymer *5 | | | | | | | 1.20 | 0.80 | 1.20 |
| Preservative | Phenoxyethanol | 0.55 | 0.55 | 0.35 | 0.55 | 0.55 | 0.55 | 0.50 | 0.55 | 0.55 |
| | Methylparaben | 0.20 | 0.20 | 0.20 | 0.20 | 0.20 | 0.20 | 0.20 | 0.20 | 0.20 |
| | Ethylparaben | 0.13 | 0.12 | 0.13 | 0.13 | 0.13 | 0.13 | 0.10 | 0.10 | 0.10 |
| Added component | 1,2-Hexanediol | 1.50 | 1.50 | 1.50 | 1.50 | 1.50 | 1.50 | | 1.50 | 1.50 |
| | Ethylhexylglycerin | 0.12 | 0.12 | 0.12 | 0.12 | 0.12 | 0.12 | 0.12 | 0.12 | 0.16 |
| | Tocopherol | 0.10 | | | | | | | | |
| | Triethanolamine | | | | | | | 0.60 | 0.40 | 0.60 |
| | Sodium dehydroacetate | | | | | | | 0.33 | | |
| Aqueous solvent | 1,3-Butylene glycol | 7.65 | 7.31 | 3.87 | 9.51 | 9.50 | 9.51 | 10.00 | 10.50 | 10.50 |
| | Water (purified water) | Balance | Balance | Balance | Balance | Balance | Balance | Balance | Balance | Balance |
| Physical properties | Viscosity mPas: 50 rpm (shear rate 192 s⁻¹) | 4.0 | 3.9 | 3.8 | 4.0 | 4.3 | 5.0 | 4.1 | 3.6 | 4.8 |
| | pH | 7.5 | 7.3 | 7.4 | 7.5 | 7.6 | 7.5 | 7.6 | 7.8 | 7.8 |
| | Surface tension (mN/m) | 35.4 | 35.4 | 35.4 | 35.4 | 35.4 | 35.4 | 35.8 | 34.3 | 31.0 |
| Evaluation | Fixation | A | A | A | A | A | A | C | B | A |
| | Sebum resistance | A | A | A | A | A | A | C | C | A |
| | Water-resistant fixation | A | A | A | A | A | A | C | B | A |
| | Drying property | B | A | B | B | A | A | C | C | B |
| | Liquid retaining property in collector type | B | B | B | B | B | B | B | B | D |

| | | | | | | | | | | |
|---|---|---|---|---|---|---|---|---|---|---|
| *1 to *5 in Table 2 above are as described below. *1: DK BLACK No. 2, available from DAITO KASEI KOGYO CO., LTD. *2: EMUPOLY-CE-119N, available from Gifu Shellac Mfg. Co., Ltd. *3: SYNTRAN EX149PE, available from INTERPOLYMER *4: SYNTRAN 5402 available from INTERPOLYMER *5: AMPHOMER HC, available from Nouryon | | | | | | | | | | |

As is clear from the results in Table 2 above, the aqueous liquid cosmetics of Examples 13 to 18, which fall within the scope of the second disclosure, are excellent in fixation, sebum resistance, water-resistant fixation, drying property, and coating property over time, as compared with the aqueous liquid cosmetics of Comparative Examples 5 to 7, which fall outside the scope of the second disclosure.

### Industrial Applicability

Provided is an aqueous liquid cosmetic that is stored in a liquid cosmetic applicator and is suitable as a makeup cosmetic such as an eyeshadow, an eyeliner, an eyebrow cosmetic, or a mascara. Reference Signs List

1 Applicator
10 Barrel body
11 Reservoir (storage portion)
12 Plug body
13 Sealing ball
14 Stopper
20 Front barrel
21 Front barrel body
23 Brush (application portion)

## Claims

1. An aqueous liquid cosmetic that is stored in a pen-type applicator having a brush at an application portion, the aqueous liquid cosmetic comprising:
at least an iron oxide pigment; a polyorganic acid or a polyorganic acid salt; emulsion particles containing acrylate copolymer; a nonionic surfactant; and an aqueous solvent.

2. The aqueous liquid cosmetic according to claim 1, wherein the polyorganic acid is selected from Group A below:
Group A: polyaspartic acid, polylactic acid, polyphosphoric acid, and polyacrylic acid.

3. The aqueous liquid cosmetic according to claim 1 or 2, wherein the emulsion particles containing acrylate copolymer are core-shell-type emulsion particles.

4. The aqueous liquid cosmetic according to claim 1 or 2, wherein the aqueous solvent is selected from water, a lower alcohol having 5 or less carbons, or a polyhydric alcohol.

5. The aqueous liquid cosmetic according to claim 1 or 2, further comprising an inorganic pigment other than the iron oxide pigment.

6. An aqueous liquid cosmetic that is stored in a type of applicator in which liquid is fed from an application liquid storage portion to an application portion using a capillary force, the aqueous liquid cosmetic comprising:
at least a pigment containing carbon as a main component; a water-soluble acrylic copolymer selected from Group X below; core-shell-type emulsion particles containing acrylate copolymer; and an aqueous solvent: Group X: (styrene/acrylate) copolymer and ammonium acrylate copolymer.

7. The aqueous liquid cosmetic according to claim 6, wherein the pigment containing carbon as a main component is selected from carbon black and/or graphite.

8. The aqueous liquid cosmetic according to claim 6 or 7, wherein the aqueous solvent is selected from water, a lower alcohol having 6 or less carbons, or a polyhydric alcohol.

9. The aqueous liquid cosmetic according to claim 8, wherein a content of the water is 50% or more.

10. The aqueous liquid cosmetic according to claim 6 or 7, wherein the application portion of the applicator is a brush.
